(19)

Europäisches Patentamt
European Patent Office
Office européen des brevets

(11) **EP 4 331 685 A1**

(12) **EUROPEAN PATENT APPLICATION**
published in accordance with Art. 153(4) EPC

(43) Date of publication:
**06.03.2024 Bulletin 2024/10**

(21) Application number: **22795766.9**

(22) Date of filing: **26.04.2022**

(51) International Patent Classification (IPC):
*A61Q 19/00* (2006.01)   *A61K 8/02* (2006.01)
*A61K 8/19* (2006.01)   *A61K 8/24* (2006.01)
*A61K 8/81* (2006.01)

(52) Cooperative Patent Classification (CPC):
**A61K 8/02; A61K 8/19; A61K 8/24; A61K 8/81;
A61Q 19/00**

(86) International application number:
**PCT/JP2022/018828**

(87) International publication number:
**WO 2022/230853 (03.11.2022 Gazette 2022/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.04.2021   JP 2021076222**

(71) Applicant: **Kao Corporation
Chuo-ku
Tokyo 103-8210 (JP)**

(72) Inventor: **SONE, Chiaki
Tokyo 131-8501 (JP)**

(74) Representative: **Hoffmann Eitle
Patent- und Rechtsanwälte PartmbB
Arabellastraße 30
81925 München (DE)**

(54) **AEROSOL COSMETIC**

(57)    An aerosol cosmetic comprising: a stock solution containing the following components (A) and (G): (A) a water-soluble thickener; and (G) water, and having a pH of 7.1 or more and 10 or less; and (C) carbonic acid gas, wherein the cosmetic has a pH of 6 or more and 7.0 or less immediately after spraying.

EP 4 331 685 A1

**Description**

Field of the Invention

The present invention relates to an aerosol cosmetic.

Background of the Invention

Skin problems such as dark circles and dullness under eyes are caused by a decrease in blood flow, and for remedying these problems, studies have been conducted on cosmetics which utilize a carbonic acid gas effect.

For example, Patent Literature 1 discloses that an aerosol cosmetic containing a combination of a specific dimethylpolysiloxane and polyethylene glycol at a specific ratio and also containing a water-soluble thickener, a specific surfactant and carbonic acid gas is unlikely to undergo volatilization of carbonic acid gas (carbon dioxide), does not make the skin sticky after application, and is excellent in use impressions such as smoothness and softness.

(Patent Literature 1) JP-A-2020-2126

Summary of the Invention

The present invention relates to an aerosol cosmetic comprising:
a stock solution containing the following components (A) and (G):

> (A) a water-soluble thickener; and
> (G) water,
> and having a pH of 7.1 or more and 10 or less; and
> (C) carbonic acid gas,
> wherein the cosmetic has a pH of 6 or more and 7.0 or less immediately after spraying.

Brief Description of Drawings

> [Figure 1] Figure 1 shows a method for measuring the rate of volatilization of carbonic acid gas and the amount of volatilization of carbonic acid gas in Examples 1 to 11 and Comparative Examples 1 and 2.
> [Figure 2] Figure 2 shows a method for measuring the concentration of carbonic acid in a cosmetic after spraying in Test Example 1.
> [Figure 3] Figure 3 shows the concentration of carbonic acid in the cosmetic immediately after spraying in Test Example 1.
> [Figure 4] Figure 4 shows the concentration of carbonic acid in the cosmetic 20 minutes after spraying in Test Example 1.
> [Figure 5] Figure 5 shows the reddishness ($\Delta$a value) of the skin when an aerosol cosmetic is applied to the skin in Test Example 2.
> [Figure 6] Figure 6 shows the lightness ($\Delta$L value) of the skin when the aerosol cosmetic is applied to the skin in Test Example 2.
> [Figure 7] Figure 7 shows the reddishness (visual observation) of the skin when the aerosol cosmetic is applied to the skin in Test Example 2.
> [Figure 8] Figure 8 shows the lightness (visual observation) of the skin when the aerosol cosmetic is applied to the skin in Test Example 2.

Detailed Description of the Invention

When a conventional aerosol cosmetic containing carbonic acid gas is applied to the skin, the blood circulation in the skin can be improved on a temporary basis, but it is difficult to make an improvement so that the skin becomes light on a sustainable basis.

The present inventors found that by adjusting a stock solution for an aerosol cosmetic to a specific pH, dissolution ability as carbonate ions is enhanced, the concentration of carbonic acid gas after spraying is increased, and volatilization is suppressed, so that it is possible to achieve a new advantage that carbonic acid gas can be fed to the skin on a sustainable basis, and thus, not only the skin is made uniformly reddish, but also even with the lapse of time, the L value remains high and the skin is kept light.

In the aerosol cosmetic of the present invention, volatilization of carbonic acid gas from froth sprayed from a container is suppressed, the concentration of carbonic acid gas in the froth is high, and carbonic acid gas can be fed to the skin on a sustainable basis. In the skin after application, increases in not only $\Delta a$ but also $\Delta L$ are high, and the skin can be improved to a uniformly reddish and light skin. Even with the lapse of time, $\Delta L$ remains high, and the lightness can be maintained.

A component (A) for use in the present invention is a water-soluble thickener, and preferably contains an anionic polymer.

The anionic polymer of the component (A) for use in the present invention may be one that is used for common cosmetics, and examples thereof include polyacrylic acid, polymethacrylic acid, acrylic acid/alkyl acrylate copolymers, carboxyvinyl polymers, carboxymethyl cellulose, carrageenan, xanthan gum, polystyrene sulfonate, agar, ghatti gum, karaya gum, pectin, alginate salts, hyaluronic acid, and alkali metal salts thereof.

Of these, acrylic acid-based polymers are preferable, acrylic acid/alkyl acrylate copolymers and carboxyvinyl polymers are more preferable, and at least one selected from the group consisting of (acrylic acid/$C_{10-30}$ alkyl acrylate) copolymers and carboxyvinyl polymers is further more preferably contained, from the viewpoint of suppressing collision between froths to retain carbonic acid gas.

Here, the (acrylic acid/$C_{10-30}$ alkyl acrylate) copolymer is a copolymer of a $C_{10-30}$ alkyl acrylate and acrylic acid, methacrylic acid or a lower alkyl ester thereof, and is crosslinked with an allyl ether of sucrose or an allyl ether of pentaerythritol. As the copolymer, commercial products such as Pemulen TR-1, Pemulen TR-2, Carbopol ETD 2020, Carbopol 1342 and Carbopol 1382 (each manufactured by Lubrizol Advanced Materials Inc.) may be used.

The water-soluble thickener other than anionic polymers may be one that is used for common cosmetics, and examples thereof include dextrin, methylcellulose, ethylcellulose, hydroxyethyl cellulose, hydroxypropyl cellulose, and polyvinyl alcohol.

One or more components (A) may be used, and the content thereof is preferably 0.1 mass% or more, more preferably 0.15 mass% or more, further more preferably 0.2 mass% or more, even more preferably 0.25 mass% or more, preferably 3 mass% or less, more preferably 2 mass% or less, further more preferably 1 mass% or less, even more preferably 0.8 mass% or less, in the stock solution, from the viewpoint of suppressing collision between froths to retain carbonic acid gas. The content of the component (A) is preferably from 0.1 to 3 mass%, more preferably from 0.15 to 2 mass%, further more preferably from 0.2 to 1 mass%, even more preferably from 0.25 to 0.8 mass%, in the stock solution.

Preferably, the stock solution further contains a (B) base.

Examples of the base as the component (B) include hydroxides of alkali metal salts such as potassium hydroxide, sodium hydroxide; hydroxides of alkaline earth metals such as magnesium hydroxide and calcium hydroxide; alkanolamines such as 2-amino-2-methyl-1-propanol, monoethanolamine, diethanolamine, and triethanolamine, and basic amino acids such as L-arginine, lysine, and ornithine; and aqueous ammonia, ammonium hydroxide, sodium hydrogen carbonate, potassium monohydrogen phosphate, and sodium monohydrogen phosphate.

As the component (B), at least one selected from the group consisting of potassium hydroxide, sodium hydroxide, potassium monohydrogen phosphate and sodium monohydrogen phosphate is preferably contained from the viewpoint that by adjusting a stock solution for an aerosol cosmetic to a specific pH, dissolution ability as carbonate ions is enhanced, the concentration of carbonic acid gas after spraying is increased, and volatilization is suppressed.

One or more components (B) may be used, and the content thereof is preferably 0.1 mass% or more, more preferably 0.15 mass% or more, further more preferably 0.17 mass% or more, even more preferably 0.2 mass% or more, and preferably 2 mass% or less, more preferably 1.5 mass% or less, further more preferably 1 mass% or less, even more preferably 0.8 mass% or less, in the stock solution, from the viewpoint that dissolution ability as carbonate ions in the stock solution for the aerosol cosmetic is enhanced, the concentration of carbonic acid gas after spraying is increased, and volatilization is suppressed. The content of the component (B) is preferably from 0.1 to 2 mass%, more preferably from 0.15 to 1.5 mass%, further more preferably from 0.17 to 1 mass%, even more preferably from 0.2 to 0.8 mass%, in the stock solution.

In the aerosol cosmetic of the present invention, the stock solution may further contain (D) an oil component which is liquid at 25°C, so that the solubility of carbonic acid gas is enhanced, the skin penetrability is improved, and the skin is improved to a uniformly reddish and light skin after application. The term "liquid" means having flowability at 25°C, and includes a paste form.

The oil component which is liquid at 25°C is not limited as long as it is used for common cosmetics, and examples thereof include silicone oils such as dimethylpolysiloxane, dimethylcyclopolysiloxane, methylphenylpolysiloxane, methylhydrogenpolysiloxane and higher alcohol-modified organopolysiloxane; hydrocarbons such as paraffin, squalane and squalene; natural animal/vegetable oils containing triglyceride as a main components, such as camellia oil, jojoba oil, olive oil, macadamia nut oil, avocado oil and olive oil; monoester oils such as glyceryl monostearate ester, glyceryl distearate ester, glyceryl monooleate ester, isopropyl palmitate, isopropyl stearate, butyl stearate, isopropyl myristate, diethyl phthalate, myristyl lactate, cetyl myristate, cetyl lactate, cetyl 2-ethylhaxanoate, 2-ethylhexyl palmitate, 2-octyldodecyl myristate, 2-octyldodecyl oleate, glycerol triisostearate, glyceryl 2-ethylhexanoate di-p-methoxy cinnamate, isotridecyl isononanoate, glyceryl monomyristate monoisostearate and alkyl benzoate (alkyl group having 12 to 15 carbon atoms); diester oils such as diisopropyl adipate, diisobutyl adipate, neopentyl glycol dicaprate, glyceryl tri(caprylate/caprate), glyceryl tricaprate, neopentyl glycol di-2-ethylhexanoate, propanediol di(caprylate/caprate), 1-isostearoyl-3-myristoyl glycerol, diisostearyl malate and di(cholesteryl/octyldodecyl) N-lauroyl-L-glutamate; ether oils such as alkyl-1,3-dimethyl butyl ether, and dicaprylyl ether; and essential oils such as eucalyptus oil and mint oil.

Of these, silicone oil, monoester oil, diester oil and triester oil are preferable, and at least liquid silicone oil is more preferably contained, from the viewpoint of enhancing the solubility of carbonic acid gas, improving the skin penetrability, and improving the skin to a uniformly reddish and light skin after application.

The liquid silicone oil is not limited as long as it is used for common cosmetics, and dimethylpolysiloxane having a viscosity of from 6 to 5,000 mPa·s at 25°C is preferably contained. The viscosity of the dimethylpolysiloxane at 25°C is preferably 10 mPa·s or more from the viewpoint of suppressing volatilization of carbonic acid gas to enhance the action on the skin, and preferably 1,000 mPa·s or less, more preferably 500 mPa·s or less, from the viewpoint of stickiness after application. The viscosity at 25°C is preferably from 10 to 1,000 mPa·s, more preferably 10 to 500 mPa·s.

Here, the viscosity is measured using a BM viscometer (manufactured by TOKISANGYO). When the viscosity is 10,000 mPa·s or less, a value obtained by performing the measurement with rotor No. 3, at 12 rpm and for 1 minute is adopted. When the viscosity is more than 10,000 mPa·s, a value obtained by performing the measurement with rotor No. 4, at 12 rpm and for 1 minute is adopted.

One or more components (D) may be used, and the content thereof is preferably 0.1 mass% or more, more preferably 0.5 mass% or more, further more preferably 1 mass% or more, and preferably 20 mass% or less, more preferably 15 mass% or less, further more preferably 10 mass% or less, in the stock solution, from the viewpoint of enhancing the solubility of carbonic acid gas, improving the skin penetrability, and improving the skin to a uniformly reddish and light skin after application. The content of the component (D) is preferably from 0.1 to 20 mass%, more preferably from 0.5 to 15 mass%, further more preferably from 1 to 10 mass%, in the stock solution.

The aerosol cosmetic of the present invention may further contain a polyhydric alcohol as a component (E) in the stock solution from the viewpoint of suppression of volatilization of carbonic acid gas and use impression after application. A polyhydric alcohol including a dihydric or trihydric alcohol is preferable.

Examples of the divalent alcohol include ethylene glycol, diethylene glycol, polyethylene glycol (number average molecular weight: less than 10,000), propylene glycol, dipropylene glycol, polypropylene glycol, propanediol, 1,3-butylene glycol, and 1,3-propanediol. Examples of the trihydric alcohol include glycerin, diglycerin, polyglycerin, and polyoxybutylene polyoxyethylene polyoxypropylene glyceryl ether (3B.O.) (8E.O.) (5P.O.) (WILBRIDE S-753 manufactured by NOF CORPORATION) . Of these, one or more selected from the group consisting of polyethylene glycol, dipropylene glycol and 1,3-propanediol is preferably contained.

One or more polyhydric alcohols as the component (E) may be used, and the content thereof is preferably 1 mass% or more, more preferably 1.5 mass% or more, further more preferably 2 mass% or more, and preferably 25 mass% or less, more preferably 20 mass% or less, further more preferably 16 mass% or less, in the stock solution, from the viewpoint of suppressing volatilization of carbonic acid gas and imparting smoothness to the skin. The content of the component (E) is preferably from 1 to 25 mass%, more preferably from 1.5 to 20 mass%, further more preferably from 2 to 16 mass%, in the stock solution.

The aerosol cosmetic of the present invention may further contain a surfactant as a component (F) in the stock solution, so that foam during spraying of carbonic acid gas is made fine to improve foam retainability.

The surfactant is not limited as long as it is used for common cosmetics, and examples thereof include nonionic surfactants, anionic surfactants, and cationic surfactants.

In the case of the nonionic surfactant, one or more nonionic surfactants having a HLB of 2 to 20 are preferably contained, and nonionic surfactants having a HLB of 2 to 10 or a HLB of 12 to 18 are preferably used alone or in combination.

Here, HLB (hydrophilic-lipophilic balance) represents a ratio of the molecular weight of the hydrophilic group moiety to the total molecular weight of the surfactant, and can be determined from the Griffin's equation in the case of the nonionic surfactant.

Examples of the nonionic surfactant having a HLB of 12 to 18 include polyglycerin fatty acid esters, polyethylene glycol fatty acid esters, sucrose fatty acid esters, polyoxyethylene fatty acid esters, polyoxyethylene sorbitan fatty acid esters, polyoxyethylene alkyl ethers, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene hydrogenated castor oil fatty acid esters, polyoxyethylene phytostanol ether, polyoxyethylene phytosterol ether, polyoxyethylene cholestanol ether, polyoxyethylene cholesteryl ether, polyoxyalkylene-modified organopolysiloxanes, and polyoxyalkylene/alkyl-co-modified organopolysiloxanes. One or more selected from these compounds may be used.

Of these, polyoxyethylene alkyl ethers and polyoxyethylene hydrogenated castor oil are preferable from the viewpoint of foaming properties during spraying of carbonic acid gas.

A content of the nonionic surfactant having a HLB of 12 to 18 is preferably from 0.05 to 10 mass%, more preferably from 0.1 to 4 mass%, further more preferably from 0.2 to 2 mass%, in the stock solution, from the viewpoint of the stability of the stock solution.

Examples of the nonionic surfactant having a HLB of 2 to 10 include sorbitan fatty acid esters having 8 to 22 carbon atoms; alkyl glyceryl ethers such as isostearyl glyceryl ether; and polyoxyalkylene-modified silicones.

Examples of the sorbitan fatty acid ester include sorbitan monopalmitate, sorbitan monostearate, sorbitan monooleate, sorbitan sesquioleate, coconut oil fatty acid sorbitan, and sorbitan tristearate. Commercial products may be used such as RHEODOL SP-P10 (HLB: 6.7) for sorbitan monopalmitate, RHEODOL SP-S10V (HLB: 4.7) for sorbitan monostearate, RHEODOL SP-O10V (HLB: 4.3) for sorbitan monooleate, RHEODOL AO-15V (HLB: 3.7) for sorbitan sesquioleate, RHEODOL SP-L10 (HLB: 8.6) for coconut oil fatty acid sorbitan, and SP-S30V (HLB: 2.1) for sorbitan tristearate (each manufactured by Kao Corporation).

Examples of the polyoxyalkylene-modified silicone include polyoxyethylene/methylpolysiloxane copolymers, and poly(oxyethylene/oxypropylene)/methylpolysiloxane copolymers. Commercial products such as "KF-6015" (PEG-3 dimethicone) (HLB: 4.5) and "KF-6019" (PEG-9 dimethicone) (HLB: 4.5) sold by Shin-Etsu Chemical Co., Ltd. and "SH-3775M" (PEG-12 dimethicone) (HLB: 5) and "SH-3773M" (PEG-12 dimethicone) (HLB: 8) sold by Dow Corning Toray Silicone Co., Ltd. may be used.

A content of the nonionic surfactant having a HLB of 2 to 10 is preferably from 0.01 to 2 mass%, more preferably from 0.05 to 1 mass%, in a stock solution.

In the present invention, as the nonionic surfactant, one or more selected from the group consisting of polyoxyethylene alkyl ethers and polyoxyethylene hydrogenated castor oil having a HLB of 12 to 18 are preferably used in combination with a polyoxyalkylene-modified silicone having a HLB of 2 to 10, from the viewpoint of foaming quality during spraying of carbonic acid gas, foam uniformity and foam wettability.

Examples of the anionic surfactant include alkyl sulfuric acid esters having 12 to 22 carbon atoms, or salts thereof, such as sodium lauryl sulfate and potassium lauryl sulfate; polyoxyethylene alkyl ether phosphoric acids having 12 to 22 carbon atoms, or salts thereof, such as sodium polyoxyethylene lauryl ether phosphate; dialkylsulfosuccinic acids having 12 to 24 carbon atoms, or salts thereof; N-alkyloylmethyltaurines having 12 to 22 carbon atoms, or salts thereof; and N-acylglutamic acids having 12 to 22 carbon atoms, or salts thereof. Of these, polyoxyethylene alkyl ether phosphoric acids or salts thereof and acylglutamic acids or salts thereof are preferable from the viewpoint of emulsion stability, and generation of a large amount of foam during spraying.

When the anionic surfactant is contained, the content thereof in the stock solution is preferably from 0.01 to 1 mass%, more preferably from 0.05 to 0.7 mass%.

The aerosol cosmetic of the present invention further contains water as a component (G) in the stock solution. The water as the component (G) serves as a solvent for the stock solution, and forms a balance in other components. A content of the water is preferably from 55 to 99 mass%, more preferably from 65 to 95 mass%, further more preferably from 70 to 90 mass%, in the stock solution, from the viewpoint of enhancing dissolution ability as carbonate ions in the stock solution and improving emulsion stability.

In the aerosol cosmetic of the present invention, the stock solution may further contain components used for common cosmetics, for example, polymers other than those described above, oil components other than those described above, ethanol, preservatives, antioxidants, pigments, pH adjusters, perfumes, ultraviolet absorbers, moisturizing agents, blood circulation promotors, cooling sensation agents, antiperspirant agents, bactericides, skin-lightening agents, anti-inflammatory agents, skin activators, and the like.

In the aerosol cosmetic of the present invention, the stock solution is prepared by mixing the components (A) and (G) and other components. A viscosity of the stock solution at 25°C is preferably from 1,000 to 10,000 mPa·s, more preferably from 1,500 to 8,500 mPa·s, further more preferably from 2,000 to 7,000 mPa·s, even more preferably from 3,000 to 7,000 mPa·s, from the viewpoint that the viscosity allows volatilization of carbonic acid foam to be suppressed, and is suitable for application to the skin.

Here, the viscosity is measured with rotor No. 3, at 12 rpm and for 1 minute using a BM viscometer (manufactured by TOKISANGYO). When the viscosity is more than 10,000 mPa·s, a value obtained by performing the measurement with rotor No. 4, at 12 rpm and for 1 minute is adopted.

In the aerosol cosmetic of the present invention, dissolution ability as carbonate ions in the stock solution for the aerosol cosmetic is enhanced, the concentration of carbonic acid gas after spraying is increased, and volatilization is suppressed. In the skin after application, increases in not only Δa but also ΔL are high, and the skin can be improved to a uniformly reddish and light skin. The pH of the stock solution is 7.1 or more and 10 or less, preferably from 7.1 to 9.5, more preferably from 7.1 to 9, from the viewpoint that the lightness is maintained with ΔL remaining high even with the lapse of time.

In the present invention, the pH of the stock solution is measured at 25°C using pH/ION-METER F-72 manufactured by HORIBA, Ltd.

The aerosol cosmetic of the present invention can be produced by filling the above-described stock solution

EP 4 331 685 A1

and (C) carbonic acid gas into a pressure-resistant container. The mode of spraying the aerosol cosmetic is preferably a foam type in which the aerosol cosmetic is sprayed in a foam form.

A mass ratio of the stock solution to the carbonic acid gas is preferably from 94 : 6 to 99.5 : 0.5, more preferably from 95 : 5 to 99 : 1, further more preferably from 96.5 : 3.5 to 98.5 : 1.5, from the viewpoint that excellent foam generation performance of carbonic acid gas is exhibited, and after application, the skin is improved to a uniformly reddish and light skin.

The aerosol cosmetic of the present invention may contain, in addition to carbonic acid gas as the component (C), another propellant which is used for common aerosol cosmetics. Examples of the propellant include liquefied petroleum gas, and compressed gas. When the aerosol cosmetic contains a propellant other than carbonic acid gas, a content of carbonic acid gas with respect to the total amount of carbonic acid gas and the propellant is preferably 85 mass% or more, more preferably 90 mass% or more, further more preferably 95 mass% or more, even more preferably 98 mass% or more.

In the present invention, the aerosol cosmetic is preferably free from a propellant other than carbonic acid gas, or when the aerosol cosmetic contains a propellant other than carbonic acid gas, the content of carbonic acid gas with respect to a total amount of carbonic acid gas and the propellant is preferably 85 mass% or more.

In the aerosol cosmetic of the present invention, the pH of the cosmetic immediately after spraying is 6 or more and 7.0 or less, preferably from 6 to 6.9, more preferably from 6.1 to 6.8, from the viewpoint that volatilization of carbonic acid gas is suppressed, the concentration of carbonic acid gas is increased, and carbonic acid gas is fed to the skin on a sustainable basis.

In the present invention, the pH of the cosmetic immediately after the spraying is measured at 25°C using pH/ION-METER F-72 manufactured by HORIBA, Ltd., with the cosmetic being filled into a glass container while being sprayed from an aerosol container. Immediately after the cosmetic is sprayed from the aerosol container, the measurement is performed, and a value of measurement after 1 minute is used.

The aerosol cosmetic of the present invention can be applied as a cosmetic without being limited, and is suitable as a skin cosmetic because volatilization of carbonic acid gas in a cosmetic after spraying is suppressed, the concentration of carbonic acid gas is high, and the effect of carbonic acid gas acting after application to the skin is high.

The aerosol cosmetic of the present invention may be used in accordance with a use situation, for example, it is applied to the skin, and left standing for a certain period of time, made compatible with the skin, wiped off, or washed off.

The aerosol cosmetic of the present invention can be produced by a normal method, for example, by the following steps 1 to 4.

Step 1: An aqueous component containing (A) an anionic polymer, (G) water and (B) a base is heated to 50°C or higher, and dispersed with a disperser.

Step 2: An oil component containing (D) an oil agent and (F) a surfactant is added to the aqueous phase obtained in step 1, and the mixture is dispersed with a disperser, and then stirred with a homomixer.

Step 3: The liquid obtained in step 2 is cooled to 15-30°C, other components such as a perfume are then added as required, and the mixture is stirred to obtain a stock solution.

Step 4: The stock solution obtained in step 3 is filled into a pressure-resistant container, the container is sealed, and a component (C) is filled under pressure.

With respect to the embodiments described above, the present invention further discloses the following compositions.

<1> An aerosol cosmetic comprising:

a stock solution containing the following components (A) and (G):

(A) a water-soluble thickener; and
(G) water,
and having a pH of 7.1 or more and 10 or less; and
(C) carbonic acid gas,

wherein the cosmetic has a pH of 6 or more and 7.0 or less immediately after spraying.

<2> The aerosol according to <1>, wherein the component (A) preferably contains an anionic polymer.
<3> The aerosol cosmetic according to <1> or <2>,
wherein the component (A) contains preferably an acrylic acid-based polymer, more preferably an acrylic acid/alkyl acrylate copolymer or a carboxyvinyl polymer, and further more preferably contains at least one selected from the group consisting of (acrylic acid/C$_{10-30}$ alkyl acrylate) copolymers and carboxyvinyl polymers.

<4> The aerosol cosmetic according to any one of <1> to <3>, wherein a content of the component (A) is preferably 0.1 mass% or more, more preferably 0.15 or more, further more preferably 0.2 mass% or more, even more preferably 0.25 mass% or more, and preferably 3 mass% or less, more preferably 2 mass% or less, further more preferably 1 mass% or less, even more preferably 0.8 or less, in the stock solution.

<5> The aerosol cosmetic according to any one of <1> to <4>, wherein the stock solution further contains (B) a base.

<6> The aerosol cosmetic according to <5>, wherein the component (B) preferably contains at least one selected from the group consisting of potassium hydroxide, sodium hydroxide, potassium monohydrogen phosphate and sodium monohydrogen phosphate.

<7> The aerosol cosmetic according to <5> or <6>, wherein a content of the component (B) is preferably 0.1 mass% or more, more preferably 0.15 mass% or more, further more preferably 0.17 mass% or more, even more preferably 0.2 mass% or more, and preferably 2 mass% or less, more preferably 1.5 mass% or less, further more preferably 1 mass% or less, even more preferably 0.8 mass% or less, in the stock solution.

<8> The aerosol cosmetic according to any one of <1> to <7>, wherein the stock solution preferably further contains (D) an oil component which is liquid at 25°C, more preferably silicone oil, monoester oil, diester oil or triester oil, further more preferably at least liquid silicone oil.

<9> The aerosol cosmetic according to <8>, wherein the liquid silicone oil is dimethylpolysiloxane having a viscosity of preferably from 6 to 5,000 mPa·s at 25°C, more preferably from 10 to 1,000 mPa·s at 25°C, further more preferably from 10 to 500 mPa·s at 25°C.

<10> The aerosol cosmetic according to <8> or <9>, wherein a content of the component (D) is preferably 0.1 mass% or more, more preferably 0.5 mass% or more, further more preferably 1 mass% or more, and preferably 20 mass% or less, more preferably 15 mass% or less, further more preferably 10 mass% or less, in the stock solution.

<11> The aerosol cosmetic according to any one of <1> to <10>, wherein the stock solution preferably further contains (E) a polyhydric alcohol, more preferably a dihydric or trihydric alcohol, further more preferably one or two selected from the group consisting of polyethylene glycol, dipropylene glycol and 1,3-propanediol.

<12> The aerosol cosmetic according to <11>, wherein a content of the component (E) is preferably 1 mass% or more, more preferably 1.5 mass% or more, further more preferably 2 mass% or more, and preferably 25 mass% or less, more preferably 20 mass% or less, further more preferably 16 mass% or less, in the stock solution.

<13> The aerosol cosmetic according to any one of <1> to <12>, wherein the stock solution preferably further contains (F) a surfactant, more preferably a nonionic surfactant, further more preferably a nonionic surfactant having a HLB of 2 to 20, and nonionic surfactants having a HLB of 2 to 10 or a HLB of 12 to 18 are even more preferably used alone or in combination.

<14> The aerosol cosmetic according to <13>, wherein the nonionic surfactant having a HLB of 12 to 18 is preferably a polyoxyethylene alkyl ether or polyoxyethylene hydrogenated castor oil, and the nonionic surfactant having a HLB of 2 to 10 is preferably a sorbitan fatty acid ester having 8 to 22 carbon atoms, an alkyl glyceryl ether or a polyoxyalkylene-modified silicone.

<15> The aerosol cosmetic according to <13> or <14>, wherein a content of the nonionic surfactant having a HLB of 12 to 18 is preferably from 0.05 to 10 mass%, more preferably from 0.1 to 4 mass%, further preferably from 0.2 to 2 mass%, in the stock solution.

<16> The aerosol cosmetic according to <13> or <14>, wherein a content of the nonionic surfactant having a HLB of 2 to 10 is preferably from 0.01 to 2 mass%, more preferably from 0.05 to 1 mass%, in the stock solution.

<17> The aerosol cosmetic according to any one of <13> to <16>, wherein as the nonionic surfactant as the component (F), one or more selected from the group consisting of polyoxyethylene alkyl ethers and polyoxyethylene hydrogenated castor oil having a HLB of 12 to 18 are preferably used in combination with a polyoxyalkylene-modified silicone having a HLB of 2 to 10.

<18> The aerosol cosmetic according to any one of <1> to <17>, wherein a content of the component (G) is preferably from 55 to 99 mass%, more preferably from 65 to 95 mass%, further more preferably from 70 to 90 mass%, in the stock solution.

<19> The aerosol cosmetic according to any one of <1> to <18>, wherein a viscosity of the stock solution at 25°C is preferably from 1,000 to 10,000 mPa·s, more preferably from 1,500 to 8,500 mPa·s, further more preferably from 2,000 to 7,000 mPa·s, even more preferably from 3,000 to 7,000 mPa·s .

<20> The aerosol cosmetic according to any one of <1> to <19>, wherein a pH of the stock solution is preferably from 7.1 to 9.5, more preferably from 7.1 to 9.

<21> The aerosol cosmetic according to any one of <1> to <20>, which is produced by filling the stock solution and (C) carbonic acid gas into a pressure-resistant container.

<22> The aerosol cosmetic according to any one of <1> to <21>, wherein a spraying mode is preferably a foam type in which the aerosol cosmetic is sprayed in a foam form.

<23> The aerosol cosmetic according to any one of <1> to <22>, wherein a mass ratio of the stock solution to carbonic acid gas is preferably from 94 : 6 to 99.5 : 0.5, more preferably from 95 : 5 to 99 : 1, further more preferably from 96.5 : 3.5 to 98.5 : 1.5.

<24> The aerosol cosmetic according to any one of <1> to <23>, wherein the aerosol cosmetic is preferably free from a propellant other than carbonic acid gas, or when the aerosol cosmetic comprises a propellant other than carbonic acid gas, a content of carbonic acid gas with respect to a total amount of carbonic acid gas and the propellant is preferably 85 mass% or more.

<25> The aerosol cosmetic according to any one of <1> to <23>, wherein the aerosol cosmetic comprises a propellant other than carbonic acid gas, the content of carbonic acid gas with respect to the total amount of carbonic acid gas and the propellant is preferably 85 mass% or more, more preferably 90 mass% or more, further more preferably 95 mass% or more, even more preferably 98 mass% or more.

<26> The aerosol cosmetic according to any one of <1> to <25>, wherein a pH of the cosmetic immediately after spraying is preferably from 6 to 6.9, more preferably from 6.1 to 6.8.

<27> The aerosol cosmetic according to any one of <1> to <26>, which is a skin cosmetic.

<28> An aerosol cosmetic comprising:

a stock solution containing the following components (A), (B) and (G) :

(A) an anionic polymer selected from the group consisting of acrylic acid-based polymers: 0.1 to 7 mass%;
(B) a base selected from the group consisting of potassium hydroxide, sodium hydroxide, potassium monohydrogen phosphate and sodium monohydrogen phosphate: 0.1 to 2 mass%; and
(G) water,
and having a pH of 7.1 or more and 10 or less; and
(C) carbonic acid gas,

wherein the cosmetic has a pH of 6 or more and 7.0 or less immediately after spraying.

<29> An aerosol cosmetic comprising:

a stock solution containing the following components (A), (B) and (G) :

(A) an anionic polymer selected from the group consisting of acrylic acid/alkyl acrylate copolymers and carboxyvinyl polymers: 0.15 to 2 mass%;
(B) a base selected from the group consisting of potassium hydroxide, sodium hydroxide, potassium monohydrogen phosphate and sodium monohydrogen phosphate: 0.15 to 1.5 mass%; and
(G) water,
and having a pH of 7.1 or more and 9.5 or less; and
(C) carbonic acid gas,

wherein the cosmetic has a pH of 6 or more and 7.0 or less immediately after spraying.

Examples

Examples 1 to 11 and Comparative Examples 1 to 2

Aerosol cosmetics were produced in accordance with the formulations shown in Tables 1 and 2. The viscosities and the pH of stock solutions at 25°C, and the pH of the cosmetics immediately after spraying were measured.
Carbonic acid gas volatilization properties, and skin colors (color measurement values and visual observations) immediately after and 3 minutes after application to the skin were evaluated.
The results thereof are collectively shown in Tables 1 and 2.

(Production method)

Step 1: An aqueous component containing (A) an aqueous thickener, (G) water, (B) a base, and polyethylene glycol was heated to 58-68°C, and dispersed with a disperser.
Step 2: An oil component containing (D) an oil agent, dipropylene glycol, 1,3-propanediol and (F) a surfactant was added to the aqueous phase obtained in step 1, and the mixture was dispersed with a disperser, and then stirred with a homomixer.

Step 3: The liquid obtained in step 2 was cooled to 15-30°C, a perfume was added, and the mixture was stirred to obtain a stock solution.

Step 4: The stock solution obtained in step 3 was filled into a pressure-resistant container, the container was sealed, and a component (C) was filled under pressure to obtain an aerosol cosmetic. To 97.6 parts by mass of the stock solution was added 2.4 parts by mass of carbonic acid gas (stock solution : carbonic acid gas = 97.6 : 2.4) .

(Evaluation method)

(1) Viscosity of stock solution:

The stock solution was filled in an amount of 50 g into a 50 mL glass container, and the viscosity was measured using a BM viscometer (manufactured by TOKISANGYO) (rotor No. 3, 12 rpm, 1 min).

(2) Stock solution pH:

The stock solution was filled in an amount of 50 g into a 50 mL glass container, and the pH was measured at 25°C using pH/10N-METER F-72 manufactured by HORIBA, Ltd.

(3) pH of cosmetic immediately after spraying:

The cosmetic was filled into a 20 mL glass container while being sprayed from an aerosol container, and the pH was measured at 25°C using pH/10N-METER F-72 manufactured by HORIBA, Ltd. Immediately after the spraying from the aerosol container, the measurement was performed, and a value of measurement after 1 minute was used.

(4) Carbonic acid gas volatilization properties:

(4-1) Rate of volatilization of carbonic acid gas

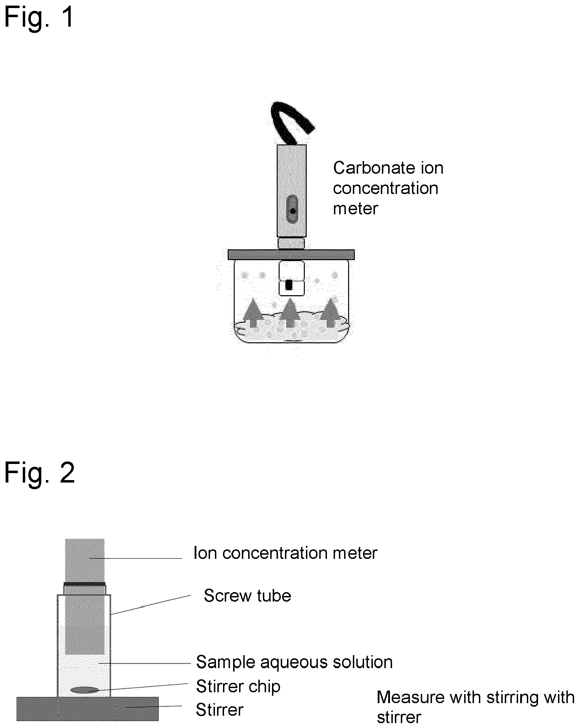

As shown in Figure 1, about 1 gf of each aerosol cosmetic was sprayed to a cylindrical glass container (volume: 19 cm$^3$), a carbon dioxide electrode CE-2041 (manufactured by DKK-TOA CORPORATION) was then inserted while being prevented from coming into contact with the cosmetic, and the concentration of carbonic acid gas was immediately measured with the glass container kept in a hermetically closed state. The measuring apparatus (carbonate ion concentration meter) is ION/pH METER IM-32P.

Immediately after spraying, the measurement started, and from the amount of change in concentration of carbonic acid gas over 4 minutes from 1 minute to 5 minutes after the start of measurement, the amount of change per minute was calculated, and taken as a rate of volatilization of carbonic acid gas (ppm/min).

(4-2) Amount of volatilization of carbonic acid gas:

In the same manner as in (4-1), the concentrations of carbonic acid gas 10 minutes after and 20 minutes after the start of measurement were determined.

(5) Evaluation of skin color (color measurement value):

A shaking cup (diameter: 3.4 cm) was attached to an inner side part of a forearm, and 0.5 g of the cosmetic was put in the shaking cup by direct spraying the cosmetic from an aerosol container, applied with a spatula so as to spread as flatly as possible, then held for 2 minutes, and then wiped off with tissue paper.

For the skin before and after application of the cosmetic, the "a" value and the "L" value are measured using a chromometer CM2600d (manufactured by KONICA MINOLTA, INC.). The measurement was repeated three times at one site, and performed immediately after and 3 minutes after removal of cosmetic.

Averages of "a" values and "L" values obtained by the three measurements are calculated. Differences between the averages and values before application, Δa and ΔL values, were calculated.

(6) Evaluation of skin color (visual observation)

During the measurement of color measurement values, the reddishness and the lightness of the skin were evaluated by visual observation on the basis of the following criteria, and an average for three tests was determined.

2: Very reddish/very light

1.5: Between being very reddish and being reddish/between being very light and being light

1: Reddish/light

0.5: Slightly reddish/slightly light

0: Equivalent to the level before application

[Table 1]

| | Component name | Raw material name | Example 1 | Example 2 | Example 3 | Example 4 | Example 5 | Example 6 | Comparative Example 1 |
|---|---|---|---|---|---|---|---|---|---|
| (A) | Acrylic acid/alkyl methacrylate copolymer | PEMULEN TR-1 manufactured by Lubrizol Corporation | 0.35 | 0.4 | 0.4 | 0.4 | 0.5 | 0.4 | 0.4 |
| (B) | Potassium hydroxide solution (48%) | | 0.612 | 0.6 | 0.7 | 0.7212 | 1.25 | | |
| (B) | Sodium hydroxide (48%) | | | | | | | 0.45 | |
| | Potassium dihydrogen phosphate | | | 0.05 | 0.2 | 0.121 | 0.5 | 0.15 | 0.15 |
| (D) | Dimethylpolysiloxane 10cs | KF-96A-10CS manufactured by Shin-Etsu Chemical Co., Ltd. | 2.02 | 2.02 | 2.02 | 2.02 | 2.02 | 2.02 | 2.02 |
| (D) | Dimethylpolysiloxane 50cs | KF-96A-50CS manufactured by Shin-Etsu Chemical Co., Ltd. | 2.0 | 2.0 | 2.0 | 2.0 | 2.0 | 2.0 | 2.0 |
| (D) | Dimethylpolysiloxane 100cs | KF-96A-100CS manufactured by Shin-Etsu Chemical Co., Ltd. | 0.5 | 0.5 | 0.5 | 0.5 | 0.5 | 0.5 | 0.5 |
| (D) | Isotridecyl isononanoate | SALACOS 913 manufactured by The Nisshin Oillio, Ltd. | 2.0 | 2.0 | 2.0 | 2.0 | 2.0 | 2.0 | 2.0 |
| (E) | Dipropylene glycol | | 5.0 | 5.0 | 5.0 | 5.0 | 5.0 | 5.0 | 5.0 |
| (E) | 1,3-Propanediol | | 2.0 | 2.0 | 2.0 | 2.0 | 2.0 | 2.0 | 2.0 |
| (E) | Polyethylene glycol 1540 | | 1.0 | 1.0 | 1.0 | 1.0 | 1.0 | 1.0 | 1.0 |
| (F) | Polyoxyethylene hydrogenated castor oil | EMANON CH-60 (K) (HLB: 14) manufactured by Kao Corporation | 0.5 | 0.5 | 0.5 | 0.5 | 0.5 | 0.5 | 0.5 |
| (F) | Polyoxyethylene/methylpolysiloxane copolymer | KF-6015 (HLB4.5) manufactured by Shin-Etsu Chemical Co., Ltd. | 0.5 | 0.5 | 0.5 | 0.5 | 0.5 | 0.5 | 0.5 |

(continued)

| | Component name | Raw material name | Example 1 | Example 2 | Example 3 | Example 4 | Example 5 | Example 6 | Comparative Example 1 |
|---|---|---|---|---|---|---|---|---|---|
| | Water dispersion of lauryl methacrylate/ ethylene glycol dimethacrylate/sodium methacrylate copolymer dispersion liquid | | 1.25 | 1.25 | 1.25 | 1.25 | 1.25 | 1.25 | 1.25 |
| | Methyl paraoxybenzoate | | 0.2 | 0.2 | 0.2 | 0.2 | 0.2 | 0.2 | 0.2 |
| | Disodium edetate | | 0.05 | 0.05 | 0.05 | 0.05 | 0.05 | 0.05 | 0.05 |
| | Phenoxyethanol | | 0.35 | 0.35 | 0.35 | 0.35 | 0.35 | 0.35 | 0.35 |
| | nicotinic acid amide | | 0.0001 | 0.0001 | 0.0001 | 0.0001 | 0.0001 | 0.0001 | 0.0001 |
| | Perfume | | 0.1 | 0.1 | 0.1 | 0.1 | 0.1 | 0.1 | 0.1 |
| (G) | Purified water | | 81.5679 | 81.4799 | 81.2299 | 81.2677 | 80.2799 | 81.5299 | 81.8299 |
| | Stock solution total | | 100.0 | 100.0 | 100.0 | 100.0 | 100.0 | 100.0 | 100.0 |
| | (A) total | | 0.35 | 0.40 | 0.40 | 0.40 | 0.50 | 0.40 | 0.40 |
| | (B) total | | 0.29 | 0.29 | 0.34 | 0.35 | 0.60 | 0.22 | 0.07 |
| | (D) total | | 6.52 | 6.52 | 6.52 | 6.54 | 6.52 | 6.52 | 6.52 |
| | | | | | | | | | |
| (C) | Carbonic acid gas | | 2.4 | 2.4 | 2.4 | 2.4 | 2.4 | 2.4 | 2.4 |
| | | | | | | | | | |
| | Viscosity of stock solution (mPa.s) | | 6090 | 6930 | 3860 | 4750 | 5050 | 4820 | 4720 |
| | pH of stock solution | | 8.47 | 7.49 | 7.42 | 8.02 | 7.94 | 7.32 | 5.06 |
| | pH of cosmetic immediately after spraying | | 6.21 | 6.15 | 6.26 | 6.30 | 6.48 | 6.21 | 5.07 |

| | Component name | Raw material name | Example 1 | Example 2 | Example 3 | Example 4 | Example 5 | Example 6 | Comparative Example 1 |
|---|---|---|---|---|---|---|---|---|---|
| Carbonic acid gas volatilization properties | | Rate of volatilization of carbonic acid gas (ppm/min) 1 to 5 minutes | 28.4 | 19.5 | 27.8 | 28.1 | 28.4 | 27.2 | 32.4 |
| | | Amount of volatilization of carbonic acid gas: concentration after 10 minutes (ppm) | 223.3 | 163.0 | 223.3 | 230.0 | 230.0 | 220.0 | 256.7 |
| | | Amount of volatilization of carbonic acid gas: concentration after 20 minutes (ppm) | 316.7 | 230.0 | 326.7 | 320.0 | 330.0 | 313.3 | 366.7 |
| Evaluation of skin color (color measurement value) | | Reddishness of skin: $\Delta a$ value (immediately after application) | 3.1 | 2.7 | 2.9 | 2.8 | 2.2 | 2.6 | 1.9 |
| | | Reddishness of skin: $\Delta a$ value (after 3 minutes) | -0.9 | -1.2 | -1.0 | -0.6 | -1.3 | -0.9 | -1.3 |
| | | Lightness of skin: $\Delta L$ value (immediately after application) | -2.0 | -1.6 | -1.8 | -1.5 | -1.1 | -1.4 | -1.7 |
| | | Lightness of skin: $\Delta L$ value (after 3 minutes) | 1.1 | 1.2 | 1.0 | 1.1 | 1.6 | 1.3 | 0.8 |
| Evaluation of skin color (visual observation) | | Reddishness of skin: visual observation score (immediately after application) | 2.0 | 2.0 | 2.0 | 2.0 | 2.0 | 2.0 | 1.5 |
| | | Lightness of skin: visual observation score (after 3 minutes) | 1.5 | 2.0 | 1.3 | 2.0 | 1.7 | 1.7 | 0.8 |

EP 4 331 685 A1

[Table 2]

| | Component name | Raw material name | Example 7 | Example 8 | Example 9 | Example 10 | Example 11 | Comparative Example 2 |
|---|---|---|---|---|---|---|---|---|
| (A) | Acrylic acid/alkyl methacrylate copolymer | PEMULEN TR-1 manufactured by Lubrizol Corporation | 0.275 | 0.6 | 0.4 | 0.4 | 0.4 | 0.4 |
| (B) | Potassium hydroxide solution (48%) | | 0.45 | 1.1 | 0.7 | 0.7 | 0.7 | 0.7 |
| | Potassium dihydrogen phosphate | | | 0.4134 | 0.1 | 0.1 | 0.1 | 0.1 |
| (D) | Methylpolysiloxane 10cs | KF-96A-10CS manufactured by Shin-Etsu Chemical Co., Ltd. | 2.02 | 2.02 | 2.02 | 2.02 | 2.02 | 2.02 |
| (D) | Methylpolysiloxane 50cs | KF-96A-50CS manufactured by Shin-Etsu Chemical Co., Ltd. | 2.0 | 2.0 | 2.0 | 2.0 | 2.0 | 2.0 |
| (D) | Methylpolysiloxane 100cs | KF-96A-100CS manufactured by Shin-Etsu Chemical Co., Ltd. | 0.5 | 0.5 | 0.5 | 0.5 | 0.5 | 0.5 |
| (D) | Isotridecyl isononanoate | SALACOS 913 manufactured by The Nisshin Oillio, Ltd. | 2.0 | 2.0 | 2.0 | 2.0 | 2.0 | 2.0 |
| (E) | Dipropylene glycol | | 5.0 | 5.0 | 5.0 | 5.0 | 5.0 | 5.0 |
| (E) | 1,3-Propanediol | | 2.0 | 2.0 | 2.0 | 2.0 | 2.0 | 2.0 |
| (E) | Polyethylene glycol 1540 | | 1.0 | 1.0 | 1.0 | 1.0 | 1.0 | 1.0 |
| (F) | Polyoxyethylene hydrogenated castor oil | EMANON CH-60 (K) (HLB:14) manufactured by Kao Corporation | 0.5 | 0.5 | 0.5 | 0.5 | 0.5 | 0.5 |
| (F) | Polyoxyethylene/methylpolysiloxane copolymer | KF-6015 (HLB4.5) manufactured by Shin-Etsu Chemical Co., Ltd. | 0.5 | 0.5 | 0.5 | 0.5 | 0.5 | 0.5 |
| | Water dispersion of lauryl methacrylate/ethylene glycol dimethacrylate/sodium methacrylate copolymer dispersion liquid | | 1.25 | 1.25 | 1.25 | 1.25 | 1.25 | 1.25 |
| | Methyl paraoxybenzoate | | 0.2 | 0.2 | 0.2 | 0.2 | 0.2 | 0.2 |

(continued)

| | Component name | Raw material name | Example 7 | Example 8 | Example 9 | Example 10 | Example 11 | Comparative Example 2 |
|---|---|---|---|---|---|---|---|---|
| | Disodium edetate | | 0.05 | 0.05 | 0.05 | 0.05 | 0.05 | 0.05 |
| | Phenoxyethanol | | 0.35 | 0.35 | 0.35 | 0.35 | 0.35 | 0.35 |
| | nicotinic acid amide | | 0.0001 | 0.0001 | 0.0001 | 0.0001 | 0.0001 | 0.0001 |
| | Perfume | | 0.1 | 0.1 | 0.1 | 0.1 | 0.1 | 0.1 |
| | Sodium hydrogen carbonate | | | | | | | 4.58 |
| (G) | Purified water | | 81.7849 | 80.3965 | 81.3099 | 81.3099 | 81.3099 | 76.7299 |
| | Stock solution total | | 100.0 | 100.0 | 100.0 | 100.0 | 100.0 | 100.0 |
| | (A) total | | 0.275 | 0.60 | 0.40 | 0.40 | 0.40 | 0.40 |
| | (B) total | | 0.22 | 0.53 | 0.34 | 0.34 | 0.34 | 0.34 |
| | (D) total | | 6.54 | 6.54 | 6.54 | 6.54 | 6.54 | 6.54 |
| | | | | | | | | |
| (C) | Carbonic acid gas | | 2.4 | 2.4 | 1.5 | 3.6 | 2.16 | |
| | LPG | | | | | | 0.24 | 5.9 |
| | | | | | | | | |
| | Viscosity of stock solution (mPa·s) | | 3890 | 8660 | 4700 | 4700 | 4980 | 0 |
| | pH of stock solution | | 8.42 | 7.60 | 8.17 | 8.17 | 8.00 | 8.14 |
| | pH of cosmetic immediately after spraying | | 6.13 | 6.29 | 6.14 | 6.16 | 6.23 | 8.66 |

EP 4 331 685 A1

(continued)

| | Component name | Raw material name | Example 7 | Example 8 | Example 9 | Example 10 | Example 11 | Comparative Example 2 |
|---|---|---|---|---|---|---|---|---|
| | Carbonic acid gas volatilization properties | Rate of volatilization of carbonic acid gas (ppm/min) 1 to 5 minutes | 29.3 | 24.0 | 23.7 | 25.9 | 29.2 | 8.0 |
| | | Amount of volatilization of carbonic acid gas: concentration after 10 minutes (ppm) | 220.0 | 184.7 | 179.0 | 197.7 | 220.0 | 50.3 |
| | | Amount of volatilization of carbonic acid gas: concentration after 20 minutes (ppm) | 303.3 | 250.0 | 253.3 | 283.3 | 283.3 | 50.7 |
| | Evaluation of skin color (color measurement value) | Reddishness of skin: $\Delta a$ value (immediately after application) | 2.74 | 2.73 | 2.42 | 2.45 | 2.73 | -0.50 |
| | | Reddishness of skin: $\Delta a$ value (after 3 minutes) | -1.24 | -0.62 | -0.83 | -1.01 | -0.89 | -0.25 |
| | | Lightness of skin: $\Delta L$ value (immediately after application) | -1.73 | -1.27 | -1.22 | -1.24 | -2.00 | 0.73 |
| | | Lightness of skin: $\Delta L$ value (after 3 minutes) | 1.87 | 1.58 | 1.20 | 1.80 | 1.22 | 0.10 |
| | Evaluation of skin color (visual observation) | Reddishness of skin: visual observation score (immediately after application) | 2.00 | 1.83 | 1.83 | 2.00 | 2.00 | 0.00 |
| | | Lightness of skin: visual observation score (after 3 minutes) | 1.50 | 1.17 | 1.17 | 1.50 | 1.33 | 0.00 |

Examples 12 to 17

In the same manner as in Examples 1 to 11, aerosol cosmetics were produced in accordance with the formulations shown in Tables 3 and 4. The viscosities and the pH of stock solutions at 25°C, and the pH of the cosmetics immediately after spraying were measured. The results are collectively shown in Tables 3 and 4.

All the cosmetics of Examples 12 to 17 are unlikely to undergo volatilization of carbonic acid gas from the cosmetic sprayed from the container, have a high concentration of carbonic acid gas, and can improve the skin to a uniformly reddish and light skin after application.

[Table 3]

| | Component name | Raw material name | Example 12 | Example 13 | Example 14 |
|---|---|---|---|---|---|
| (A) | Acrylic acid/alkyl methacrylate copolymer | PEMULEN TR-1 manufactured by Lubrizol Corporation | - | 0.3 | 0.4 |
| (A) | Carboxyvinyl polymer | CARBOPOL 981 POLYMER manufactured by Lubrizol Corporation | 0.4 | - | |
| (B) | Potassium hydroxide solution (48%) | | 0.6 | 0.45 | 0.631 |
| | Potassium dihydrogen phosphate | | 0.05 | 0.0375 | |
| (B) | Sodium monohydrogen phosphate | | | | 0.3014 |
| | Sodium dihydrogen phosphate | | | | 0.0169 |
| (D) | Dimethylpolysiloxane 10cs | KF-96A-10CS manufactured by Shin-Etsu Chemical Co., Ltd. | 2.02 | 2.02 | 2.02 |
| (D) | Dimethylpolysiloxane 50cs | KF-96A-50CS manufactured by Shin-Etsu Chemical Co., Ltd. | 2.0 | 2.0 | 2.0 |
| (D) | Dimethylpolysiloxane 100cs | KF-96A-100CS manufactured by Shin-Etsu Chemical Co., Ltd. | 0.5 | 0.5 | 0.5 |
| (D) | Isotridecyl isononanoate | SALACOS 913 manufactured by The Nisshin Oillio, Ltd. | 2.0 | 2.0 | 2.0 |
| (E) | Dipropylene glycol | | 5.0 | 5.0 | 5.0 |
| (E) | 1,3-Propanediol | | 2.0 | 2.0 | 2.0 |
| (E) | Polyethylene glycol 1540 | | 1.0 | 1.0 | 1.0 |
| (F) | Polyoxyethylene hydrogenated castor oil | EMANON CH-60 (K) (HLB: 14) manufactured by Kao Corporation | 0.5 | 0.5 | 0.5 |
| (F) | Polyoxyethylene/methylpolysiloxane copolymer | KF-6015 (HLB4.5) manufactured by Shin-Etsu Chemical Co., Ltd. | 0.5 | 0.5 | 0.5 |
| (A) | Xanthan gum | | - | 0.05 | - |
| | Water dispersion of lauryl methacrylate/ ethylene glycol dimethacrylate/sodium methacrylate copolymer dispersion liquid | | 1.25 | 1.25 | - |

(continued)

| | Component name | Raw material name | Example 12 | Example 13 | Example 14 |
|---|---|---|---|---|---|
| | Anhydrous silicic acid | SUNSPHERE NP-30 manufactured by AGC Inc. | | | 0.1 |
| | Methyl paraoxybenzoate | | 0.2 | 0.2 | 0.2 |
| | Disodium edetate | | 0.05 | 0.05 | 0.05 |
| | Phenoxyethanol | | 0.35 | 0.35 | 0.35 |
| | nicotinic acid amide | | 0.0001 | 0.0001 | - |
| | Perfume | | 0.1 | 0.1 | 0.1 |
| | Extract of ginseng | | 0.1 | 0.1 | 0.1 |
| (G) | Purified water | | 81.3799 | 81.5924 | 82.2307 |
| | Stock solution total | | 100.00 | 100.00 | 100.00 |
| | (A) total | | 0.40 | 0.35 | 0.40 |
| | (B) total | | 0.29 | 0.22 | 0.60 |
| | (D) total | | 6.52 | 6.52 | 6.52 |
| | | | | | |
| (C) | Carbonic acid gas | | 2.4 | 2.4 | 2.4 |
| | | | | | |
| | Viscosity of stock solution (mPa·s) | | 4280 | 5310 | 4570 |
| | pH of stock solution | | 7.60 | 7.50 | 8.10 |
| | pH of cosmetic immediately after spraying | | 6.20 | 6.11 | 6.27 |

[Table 4]

| | Component name | Raw material name | Example 15 | Example 16 | Example 17 |
|---|---|---|---|---|---|
| (A) | Acrylic acid/alkyl methacrylate copolymer | PEMULEN TR-1 manufactured by Lubrizol Corporation | 0.35 | 0.35 | 0.4 |
| (B) | Potassium hydroxide solution (48%) | | 0.575 | 0.6 | 0.6 |
| | Potassium dihydrogen phosphate | | 0.08 | 0.05 | 0.05 |
| (B) | L-arginine | L-Arginine Grade C | | | 0.2 |
| (D) | Methylpolysiloxane 10cs | KF-96A-10CS manufactured by Shin-Etsu Chemical Co., Ltd. | 2.02 | 2.02 | 2.02 |
| (D) | Methylpolysiloxane 50cs | KF-96A-50CS manufactured by Shin-Etsu Chemical Co., Ltd. | 2.0 | 2.0 | 2.0 |
| (D) | Methylpolysiloxane 100cs | KF-96A-100CS manufactured by Shin-Etsu Chemical Co., Ltd. | 0.5 | 0.5 | 0.5 |

(continued)

| | | Component name | Raw material name | Example 15 | Example 16 | Example 17 |
|---|---|---|---|---|---|---|
| | (D) | Isotridecyl isononanoate | SALACOS 913 manufactured by The Nisshin Oillio, Ltd. | 2.0 | 2.0 | 2.0 |
| | (E) | Dipropylene glycol | | 5.0 | 5.0 | 5.0 |
| | (E) | 1,3-Propanediol | | 2.0 | 2.0 | 2.0 |
| | (E) | Polyethylene glycol 1540 | | 1.0 | 1.0 | 1.0 |
| | (E) | Polyethylene glycol 400 | | 0.1 | | |
| | (F) | Polyoxyethylene hydrogenated castor oil | EMANON CH-60 (K) (HLB: 14) manufactured by Kao Corporation | 0.5 | 0.5 | 0.5 |
| | (F) | Polyoxyethylene/methylpolysiloxane copolymer | KF-6015 (HLB45) manufactured by Shin-Etsu Chemical Co., Ltd. | 0.5 | 0.5 | 0.5 |
| | (A) | Hydroxyethyl cellulose | DICEL SE900 manufactured by Dice! Miraizu Ltd. | | 0.1 | |
| | | Water dispersion of lauryl methacrylate/ ethylene glycol dimethacrylate/sodium methacrylate copolymer dispersion liquid | | | 1.25 | 1.25 |
| | | Anhydrous silicic acid | SUNSPHERE NP-30 manufactured by AGC Inc. | 0.1 | | |
| | | Methyl paraoxybenzoate | | | 0.2 | 0.2 |
| | | Disodium edetate | | 0.05 | 0.05 | 0.05 |
| | | Phenoxyethanol | | 0.35 | 0.35 | 0.35 |
| | | nicotinic acid amide | | | 0.0001 | 0.0001 |
| | | Perfume | | 0.1 | 0.1 | 0.1 |
| | | Extract of ginseng | | 0.1 | 0.1 | 0.1 |
| | | Extract of eucalyptus | | 0.0001 | | |
| | (G) | Purified water | | 82.675 | 81.3299 | 81.1799 |
| | | Stock solution total | | 100.0 | 100.00 | 100.00 |
| | | (A) total | | 0.35 | 0.45 | 0.40 |
| | | (B) total | | 0.28 | 0.29 | 0.49 |
| | | (D) total | | 6.52 | 6.52 | 6.52 |
| | | | | | | |
| | (C) | Carbonic acid gas | | 2.4 | 2.4 | 2.4 |
| | | | | | | |
| | | Viscosity of stock solution (mPa.s) | | 5100 | 4280 | 4280 |
| | | pH of stock solution | | 8.12 | 7.60 | 7.60 |
| | | pH of cosmetic immediately after spraying | | 6.20 | 6.20 | 6.20 |

Test Example 1

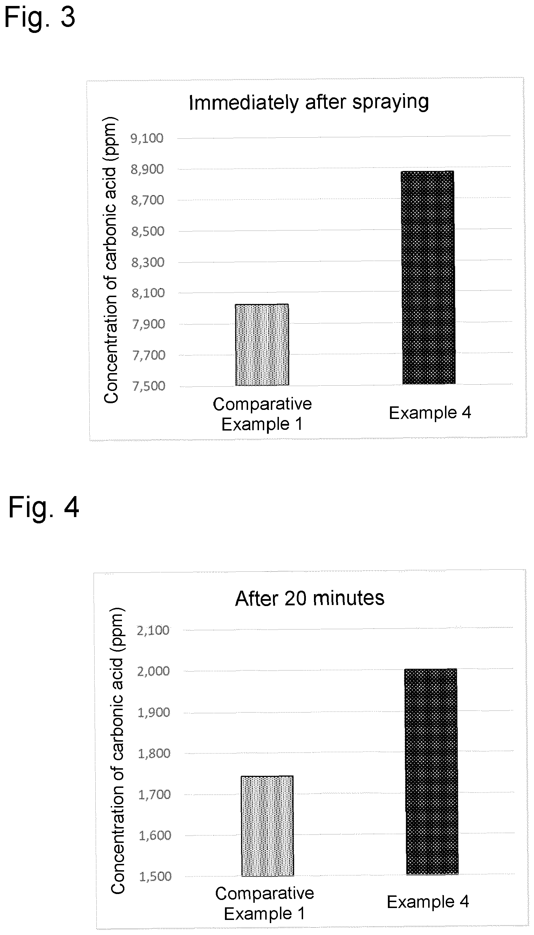

For the aerosol cosmetics of Example 4 and Comparative Example 1, the concentrations of carbonic acid in the cosmetic immediately after and 20 minutes after spraying were measured. The results are shown in Figures 3 and 4.

(Method for measuring concentration of carbonic acid)

As shown in Figure 2, 30 g of purified water was accurately weighed and taken in a screw tube, and two drops of a 48% aqueous KOH solution were added. The amount of the 48% KOH was correctly recorded. About 1.0 g of the cosmetic was accurately weighed, and directly sprayed into the screw tube from an aerosol container, and the mixture was stirred with a stirrer for 5 minutes. Thereafter, 2.5 g of 20% citric acid was accurately weighed and taken therein, and the concentration of carbonic acid was immediately measured using an ion concentration meter.
The measurement was performed while the sample aqueous solution was stirred with a stirrer.
The concentration of carbonic acid contained in the cosmetic was calculated by applying the result of measurement with the ion concentration meter to the following equation:

$$\text{concentration of carbonic acid in cosmetic (ppm)} =$$
$$\text{measured value (ppm)} \times \{\text{weight of cosmetic (g)} / (\text{weight}$$
$$\text{of cosmetic (g)} + \text{weight of water (g)} + \text{weight of 48\% KOH}$$
$$\text{(g)} + \text{weight of 20\% citric acid (g)})\}.$$

The results in Figures 3 and 4 demonstrate that the cosmetic of Example 4 had a higher concentration of carbonic acid over Comparative Example 1 immediately after and 20 minutes after spraying.

Test Example 2

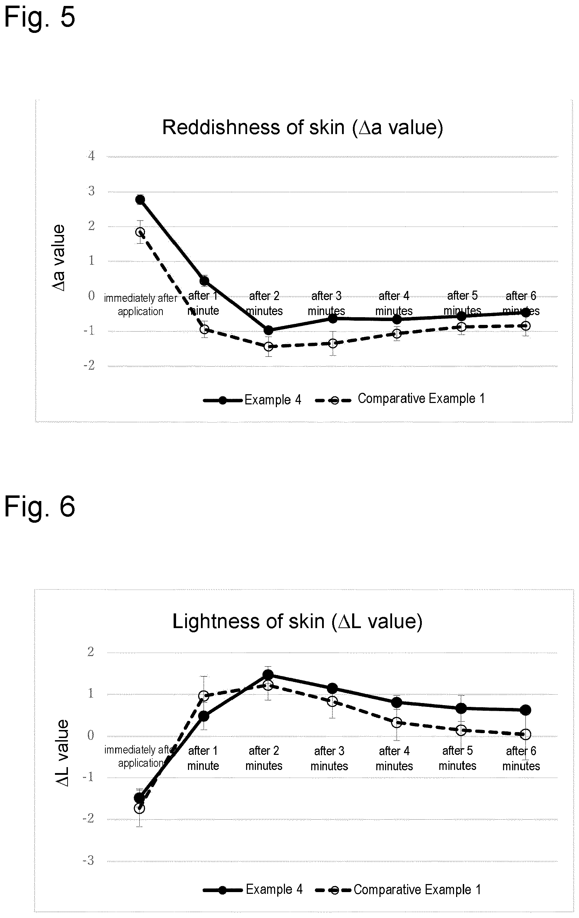

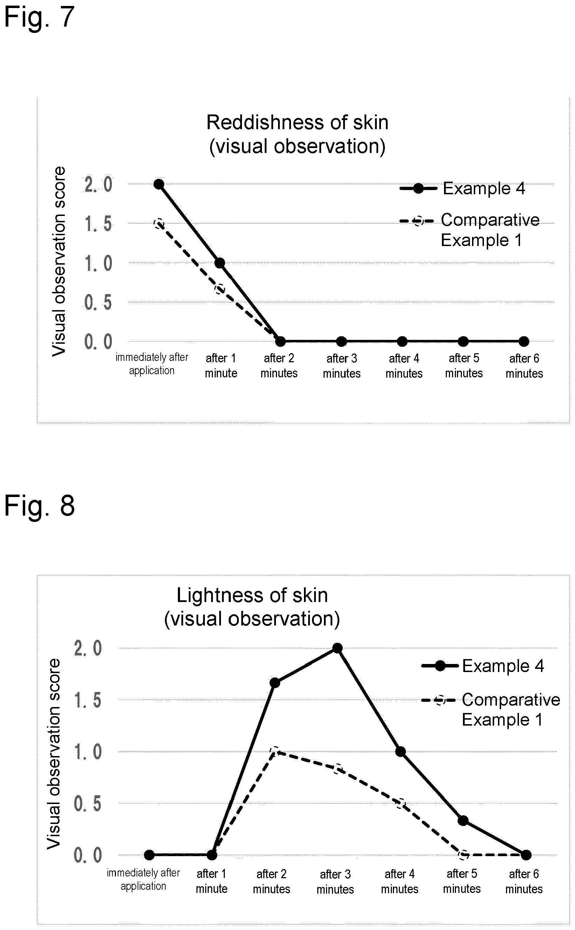

For the aerosol cosmetics of Example 4 and Comparative Example 1, the reddishness of the skin and the lightness of the skin (color measurement value and visual observation) when the cosmetic is applied to the skin are evaluated. The results are shown in Figures 5 to 8.

(Evaluation method)

A shaking cup (diameter: 3.4 cm) was attached to an inner side part of a forearm, and 0.5 g of the cosmetic was put in the shaking cup by direct spraying the cosmetic from an aerosol container, applied with a spatula so as to spread as flatly as possible, then held for 2 minutes, and then wiped off with tissue paper.

(i) Color measurement value

For the skin before and after application of the cosmetic, the "a" value and the "L" value are measured using a chromometer CM2600d (manufactured by KONICA MINOLTA, INC.). The measurement was repeated three times at one site. After removal of the cosmetic, the measurement was performed at intervals of 1 minute for up to 5 minutes after the removal.
Averages of "a" values and "L" values obtained by the three measurements are calculated. Differences between the averages and values before application, $\Delta a$ and $\Delta L$ values, were calculated.

(ii) Visual observation

During the measurement of color measurement values, the reddishness and the lightness of the skin were evaluated by visual observation on the basis of the following criteria, and an average for three tests was determined.

2: Very reddish/very light
1.5: Between being very reddish and being reddish/between being very light and being light
1: Reddish/light
0.5: Slightly reddish/slightly light

0: Equivalent to the level before application

The results in Figures 5 to 8 demonstrate that as compared to the aerosol cosmetic of Comparative Example 1, the aerosol cosmetic of Example 4, when applied to the skin, allows increases in not only $\Delta L$ but also $\Delta a$ to remain high for a long period of time, and can improve the skin to a uniformly reddish and light skin. In particular, a light skin can be maintained.

**Claims**

1.  An aerosol cosmetic comprising:

    a stock solution containing the following components (A) and (G):

    (A) a water-soluble thickener; and
    (G) water,
    and having a pH of 7.1 or more and 10 or less; and
    (C) carbonic acid gas,

    wherein the cosmetic has a pH of 6 or more and 7.0 or less immediately after spraying.

2.  The aerosol cosmetic according to claim 1, wherein the component (A) comprises an anionic polymer.

3.  The aerosol cosmetic according to claim 2, wherein the component (A) comprises at least one selected from the group consisting of (acrylic acid/$C_{10-30}$ alkyl acrylate) copolymers and carboxyvinyl polymers.

4.  The aerosol cosmetic according to any one of claims 1 to 3, comprising the component (A) in an amount of from 0.1 to 3 mass% in the stock solution.

5.  The aerosol cosmetic according to any one of claims 1 to 4, wherein the stock solution further comprises (B) a base.

6.  The aerosol cosmetic according to claim 5, wherein the component (B) comprises at least one selected from the group consisting of potassium hydroxide, sodium hydroxide, potassium monohydrogen phosphate and sodium monohydrogen phosphate.

7.  The aerosol cosmetic according to claim 5 or 6, comprising the component (B) in an amount of from 0.1 to 2 mass% in the stock solution.

8.  The aerosol cosmetic according to any one of claims 1 to 7, wherein the stock solution further comprises (D) an oil component which is liquid at 25°C.

9.  The aerosol cosmetic according to any one of claims 1 to 8, wherein the stock solution further comprises (E) a polyhydric alcohol.

10.  The aerosol cosmetic according to any one of claims 1 to 9, wherein the stock solution further comprises (F) a surfactant.

11.  The aerosol cosmetic according to any one of claims 1 to 10, wherein the stock solution has a viscosity of from 1,000 to 10,000 mPa·s at 25°C.

12.  The aerosol cosmetic according to any one of claims 1 to 11, wherein a mass ratio of the stock solution to carbonic acid gas is from 94 : 6 to 99.5 : 0.5.

13.  The aerosol cosmetic according to any one of claims 1 to 12, wherein the aerosol cosmetic is free from a propellant other than carbonic acid gas, or when the aerosol cosmetic comprises a propellant other than carbonic acid gas, a content of carbonic acid gas with respect to a total amount of carbonic acid gas and the propellant is 85 mass% or more.

## Fig. 1

Carbonate ion
concentration
meter

## Fig. 2

Ion concentration meter

Screw tube

Sample aqueous solution

Stirrer chip

Stirrer

Measure with stirring with stirrer

Fig. 3

Immediately after spraying

Concentration of carbonic acid (ppm)

9,100
8,900
8,700
8,500
8,300
8,100
7,900
7,700
7,500

Comparative
Example 1

Example 4

Fig. 4

After 20 minutes

Concentration of carbonic acid (ppm)

2,100
2,000
1,900
1,800
1,700
1,600
1,500

Comparative
Example 1

Example 4

Fig. 5

Reddishness of skin (Δa value)

Example 4　　　Comparative Example 1

Fig. 6

Lightness of skin (ΔL value)

Example 4　　　Comparative Example 1

Fig. 7

Reddishness of skin
(visual observation)

Fig. 8

Lightness of skin
(visual observation)

## INTERNATIONAL SEARCH REPORT

| International application No. |
|---|
| **PCT/JP2022/018828** |

**A.    CLASSIFICATION OF SUBJECT MATTER**

*A61Q 19/00*(2006.01)i; *A61K 8/02*(2006.01)i; *A61K 8/19*(2006.01)i; *A61K 8/24*(2006.01)i; *A61K 8/81*(2006.01)i
FI:    A61K8/02; A61K8/81; A61K8/19; A61K8/24; A61Q19/00

According to International Patent Classification (IPC) or to both national classification and IPC

**B.    FIELDS SEARCHED**

Minimum documentation searched (classification system followed by classification symbols)

A61Q19/00; A61K8/02; A61K8/19; A61K8/24; A61K8/81

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | JP 2020-007280 A (KAO CORP.) 16 January 2020 (2020-01-16) | 1, 4, 8-13 |
|  | examples 7, 8, claims, paragraphs [0030], [0033], [0041] |  |
| A |  | 2-3, 5-7 |
| X | JP 11-228334 A (KAO CORP.) 24 August 1999 (1999-08-24) | 1-13 |
|  | examples 1-3, the product of the present inventions 1-15, claims, paragraphs [0007]-[0010], [0022]-[0027], [0029] |  |
| Y |  | 1-13 |
| X | JP 2019-104766 A (KAO CORP.) 27 June 2019 (2019-06-27) | 1-13 |
|  | examples 1-29, claims |  |
| X | JP 2020-002126 A (KAO CORP.) 09 January 2020 (2020-01-09) | 1-13 |
|  | examples 1-9, prescription examples 1, 2, claims, paragraph [0007] |  |
| X | JP 2020-200282 A (KAO CORP.) 17 December 2020 (2020-12-17) | 1-13 |
|  | examples 1-9, claims |  |

☑ Further documents are listed in the continuation of Box C.          ☑ See patent family annex.

| | |
|---|---|
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **10 June 2022** | **05 July 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
|---|
| **PCT/JP2022/018828** |

**C.    DOCUMENTS CONSIDERED TO BE RELEVANT**

| Category* | Citation of document, with indication, where appropriate, of the relevant passages | Relevant to claim No. |
|---|---|---|
| X | CN 110882184 A (SHANGHAI CAL QIDONG DAILY CHEMICAL CO., LTD.) 17 March 2020 (2020-03-17)<br>      examples 1-5, claims | 1-13 |
| X | JP 2009-191042 A (POLA CHEM. IND., INC.) 27 August 2009 (2009-08-27)<br>      examples 1-4, claims, paragraph [0014] | 1, 4-11 |
| A | | 2-3, 12-13 |
| Y | JP 2005-179262 A (KAO CORP.) 07 July 2005 (2005-07-07)<br>      products of the present invention 3, 4, claims, paragraphs [0018], [0023], [0028], [0030], [0033] | 1-13 |
| A | JP 2003-252725 A (DAIZO KK) 10 September 2003 (2003-09-10)<br>      claims, examples | 1-13 |
| A | JP 2009-292740 A (POLA CHEM. IND., INC.) 17 December 2009 (2009-12-17)<br>      claims, examples | 1-13 |

Form PCT/ISA/210 (second sheet) (January 2015)

INTERNATIONAL SEARCH REPORT
Information on patent family members

International application No.

**PCT/JP2022/018828**

| Patent document cited in search report | | | Publication date (day/month/year) | Patent family member(s) | Publication date (day/month/year) |
|---|---|---|---|---|---|
| JP | 2020-007280 | A | 16 January 2020 | (Family: none) | |
| JP | 11-228334 | A | 24 August 1999 | US 6228378 B1<br>examples 1-3, products of the present invention 1-15, claims, column 1, lines 40-46, column 1, line 58 to column 2, line 42, column 3, line 56 to column 4, line 67<br>EP 920851 A2 | |
| JP | 2019-104766 | A | 27 June 2019 | (Family: none) | |
| JP | 2020-002126 | A | 09 January 2020 | CN 112203637 A<br>examples 1-9, prescription example 1, 2, claims, paragraph [0016]<br>WO 2019/245024 A1<br>KR 10-2021-0002586 A | |
| JP | 2020-200282 | A | 17 December 2020 | (Family: none) | |
| CN | 110882184 | A | 17 March 2020 | (Family: none) | |
| JP | 2009-191042 | A | 27 August 2009 | (Family: none) | |
| JP | 2005-179262 | A | 07 July 2005 | (Family: none) | |
| JP | 2003-252725 | A | 10 September 2003 | (Family: none) | |
| JP | 2009-292740 | A | 17 December 2009 | (Family: none) | |

Form PCT/ISA/210 (patent family annex) (January 2015)

**EP 4 331 685 A1**

**Patent documents cited in the description**

- JP 2020002126 A